(19) Europäisches Patentamt
European Patent Office
Office européen des brevets

(11) Publication number: **0 001 126**
**B1**

(12) **EUROPEAN PATENT SPECIFICATION**

(45) Date of publication of patent specification: **18.08.82**

(51) Int. Cl.³: **C 07 F 15/00,** A 61 K 31/28, A 61 K 31/70

(21) Application number: **78100871.9**

(22) Date of filing: **12.09.78**

(54) **Cis-platinum (II) complexes of 1,2-diamino-cyclohexane and pharmaceutical composition containing them.**

(30) Priority: **12.09.77 JP 108921/77**

(43) Date of publication of application:
**21.03.79 Bulletin 79/6**

(45) Publication of the grant of the patent:
**18.08.82 Bulletin 82/33**

(84) Designated Contracting States:
**DE FR GB**

(56) References cited:
**CHEMICAL ABSTRACTS, vol. 87, 1977, 15694z. HALL, L.M. et al. "Analogs of sulfato 1,2-diaminocyclohexane platinum (II) (SHP). II. Modifications other than the leaving ligand".**

The file contains technical information submitted after the application was filed and not included in this specification.

(73) Proprietor: **Kidani, Yoshinori**
**2-1, Mataho-cho Nishi-ku**
**Nagoya-shi Aichi-ken (JP)**

(72) Inventor: **Kidani, Yoshinori**
**2-1, Mataho-cho Nishi-ku**
**Nagoy-shi Aichi-ken (JP)**
Inventor: **Noji, Masahide**
**184-5 Oaza-Kikko-Aza-Fukazawa Moriyama-ku**
**Nagoya-shi Aichi-ken (JP)**

(74) Representative: **Casalonga, Alain et al,**
**BUREAU D.A. CASALONGA OFFICE JOSSE & PETIT Baaderstrasse 12-14**
**D-8000 München 5 (DE)**

**EP 0 001 126 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Cis-platinum (II) complexes of 1,2-diamino-cyclohexane and pharmaceutical composition containing them

The present invention relates to new platinum (II) complexes. Heretofore, many reports have been made about platinum (II) complexes and the anti-tumor activity thereof, e.g. (Chem.Biol. Interaction, vol.5 415—424 (1972), Bioinorg.Chem. vol.*2*, 187—210 (1973), Res.Commun.Chem.Pathol. Pharmacol. vol.*7*, 529—538, etc.). The present inventors, while studying cis-platinum (II) complexes of 1,2-diamino-cyclohexane (hereinafter referred to as "dac"), succeeded to resolve dac, which is used as a starting compound, into three isomers, i.e., cis-, trans--, and trans-d-; synthesized cis-platinum (II) complexes using these isomers and found that these complexes have anti-tumor activity. These complexes, their synthesis and utility are disclosed in JAP Patent Application No. 31648/78.

These three isomers of dac and their synthesis are disclosed in R.Saito and Y.Kidani; Chemistry Letters, 123—126, (1976), K.Kidani et al, J. Clin. Hemat. and Oncol 7 (1) 197—209 (1977) which was published in January 1977.

Further the above cis-platinum (II) complexes especially cis-Pt(cis-, trans-d, or trans-l-dac)$Cl_2$, their synthesis and utility are disclosed in Ito, Fujita, Saito; Bull. of the Chem. Society of Japan *40*, 2584—2591 (1967), the published draft for the lectures in the 34th spring annual meeting of the Chemical Society of Japan, 39 (1976) and the published summary of the lectures given during the 96th annual meeting of Pharmaceutical Society of Japan 79 (1976). Cis-Pt (II) (trans-l,d-dac)$SO_4$ is disclosed in L.M.Hall et al., J. Clin. Hemat. and Oncol. *7*, (1), 231—241 (1977).

It has now been found that various other cis-platinum (II) complexes may be synthetized using the three isomers of dac as a starting compound; and these complexes exhibit strong anti-tumor activity.

In accordance with the present invention, novel platinum (II) complexes represented by the general formula (I)

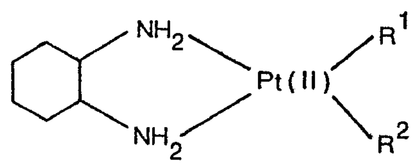

wherein $R^1$ and $R^2$ are the same or different and represent $NO_3^-$,

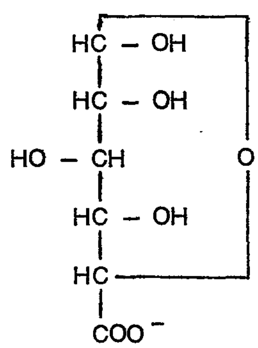

provided that $R^1$ and $R^2$ are not $NO_3^-$ at the same time or $R^1$ and $R^2$ combine to form $SO_4^{--}$ and the stereoisomerism of 1,2-diaminocyclohexane is cis-, trans-l- or trans-d- provided that when $R^1$ and $R^2$ combine to form $SO_4^{--}$ the stereoisomerism of 1,2-diaminocyclohexane is trans-l. The complexes of the present invention have anti-tumor activity and are, therefore, useful as carcinostatic agents.

The novel complexes of the invention are prepared by applying known methods, such as the methods disclosed in Journal of Pharmaceutical Sciences *65*, 315—328 (1976). For example

(1) Pt(II) (dac)$X_2$ is prepared by reacting $K_2PtX_4$ (X represents halogen) with dac.

(2) Pt(II) (dac)$(NO_3)_2$ and Pt (II) (dac) $SO_4$ are prepared by reacting Pt(II) (dac)$X_2$ with silver nitrate and silver sulfate respectively.

(3) The glucuronate of Pt(II) (dac) is prepared by reacting Pt(II)(dac)$(NO_3)_2$ obtained by method (2) with sodium glucuronate.

The above reactions are carried out in water, if necessary, with heating (about 90°C) or under interception of light. The reactions are usually completed in 3 to 48 hours to form generally white to yellow precipitates. The precipitates are recrystallized from 0.1N HCl, or the like, whereby the desired products may be obtained in crystalline form.

This method is also described in the above-identified JAP Patent Application 31648/78.

Diamines used in the present invention are cis-, trans-l- or trans-d- isomers of dac. Processes for

the preparation of the three isomers of dac are described in the above-mentioned JAP Patent Application 31648/78 and in the above-mentioned articles in Chemistry Letters and J.Clin. Hemat. and Onc.

Representative compounds of the cis-platinum (II) complex of the present invention and their elementary analysis values are set forth respectively in the following Tables 1 and 2.

TABLE 1

| Compound No. | Compound | Example No. |
|---|---|---|
| 1 | cis-Pt(trans-l-dac)$SO_4$ | 1 |
| 2 | cis-Pt(cis-dac)(D–GlucH)$NO_3$·$2H_2O$ | 2 |
| 3 | cis-Pt(trans-l-dac)(D–GlucH)$NO_3$·$2H_2O$ | 2 |
| 4 | cis-Pt(trans-d-dac)(D–GlucH)$NO_3$·$2H_2O$ | 2 |
| 5 | cis-Pt(cis-dac)(D–GlucH)$_2$·$3H_2O$ | 3 |
| 6 | cis-Pt(trans-l-dac)(D–GlucH)$_2$·$3H_2O$ | 3 |
| 7 | cis-Pt(trans-d-dac)(D–GlucH)$_2$·$3H_2O$ | 3 |

In the foregoing Table and elsewhere herein the following abbreviation is used:

GlucH :

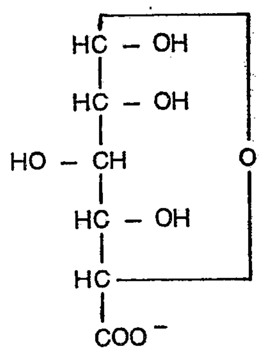

TABLE 2

Elementary Analysis

| Compound No. | Found (%) C | Found (%) H | Found (%) N | Calculated (%) C | Calculated (%) H | Calculated (%) N |
|---|---|---|---|---|---|---|
| 1 | 17.93 | 3.67 | 6.79 | 17.78 | 3.48 | 6.91 |
| 2 | 23.21 | 4.45 | 6.82 | | | |
| 3 | 23.45 | 4.72 | 6.95 | 24.00 | 4.53 | 7.00 |
| 4 | 23.18 | 4.60 | 7.03 | | | |
| 5 | 27.27 | 4.55 | 3.45 | | | |
| 6 | 28.25 | 4.67 | 3.58 | 28.84 | 5.11 | 3.74 |
| 7 | 27.80 | 4.60 | 3.52 | | | |

Cis-platinum (II) complexes of the present invention exhibit anti-tumor activity upon experimental tumors induced in mice, such as P388, S180A and L1210; and, therefore, are useful as chemotherapeutic carcinostatic agents.

The cis-platinum (II) complexes also exhibit anti-microbial activity and are, therefore, also useful as disinfectants for cleaning and sterilizing surfaces and the like.

As chemotherapeutic agents, the cis-platinum (II) complexes of the invention may be administered orally, intramuscularly or intravenously. They may be formulated as capsules, powders, pellets or injections in combination with various known pharmaceutical lubricants, binders and excipients.

Suitable dosage of the cis-platinum (II) complexes is about 1 to 400 mg/kg/day in animals.

The anti-tumor activity of the present cis-platinum (II) complexes is illustrated by the following representative experiments.

Experiment 1

In this experiment the activity against experimental tumor P388 in mice is tested.

Leukemia P388 cells were injected intraperitoneally to groups of $CDF_1$ mice (Number of transplanted cells was $10^6$ per mouse); and on the first and fifth days after transplantation, the test compound was administered by intraperitoneal injection to the mice. The dosage and test compound are set forth in the following Tables 3 and 4.

The effect of the test compound was evaluated by means of extension of average survival period T/C % (average survival days of the groups administered with the test compound/average survival days of the control group). When the T/C % is equal to or more that 120%, the test compound is considered to be effective. The results are also shown in Tables 3 and 4.

TABLE 3

| Compound No. | Toxic Dose (mg/kg) | Optimum Dose (mg/kg) | Optimum Dose T/C (%) | MED (mg/kg) | MED T/C (%) | T.I. |
|---|---|---|---|---|---|---|
| 1 | 12.5 | 6.25 | 212 | 1.56 | 140 | 4 |
| 2 | 50 | 25 | 208 | 1.56 | 139 | 16 |
| 3 | 50 | 25 | 203 | 0.39 | 124 | 64 |
| 4 | 50 | 25 | 188 | 1.56 | 120 | 16 |
| 5 | ≧200 | 100 | 191 | 6.25 | 134 | ≧16 |
| 6 | 100 | 50 | 224 | 3.12 | 141 | 16 |
| 7 | 100 | 50 | 181 | 6.25 | 123 | 8 |

MED: The lowest dose where T/C % exceeds 120 %

T.I.: Therapeutic Index (Optimum Dose / MED)

TABLE 4

| Compound No. | Dose (mg/kg) 25 | 12.5 | 6.25 |
|---|---|---|---|
| 1 | 0 | 83 | 212 |
| 2 | 208 | 184 | 170 |
| 3 | | 203 | 187 |
| 4 | 188 | 180 | 147 |
| 5 | 178 | 153 | 134 |
| 6 | 189 | 176 | 147 |
| 7 | 170 | 133 | 123 |

Experiment 2

In this experiment the activity against experimental tumor ascites-type S180A in mice is tested.

Ascites-type sarcoma 180A were transplanted intraperitoneally to ddN mice. The test compound was then interperitoneally injected every day from one to five days. The resulting ascites in the mice were sampled to evaluate the anti-tumor activity of the test compound by means of the growth rate of the tumor, T/C % (groups administered with the test compound/control group).

The results are shown in Table 5 below wherein the designation "+++" means 10—0% of the T/C % respectively and the term "toxic" is used in the case where all the mice died.

TABLE 5

| Compound No. | Dose (mg/kg) | T/C (%) | Effect |
|---|---|---|---|
| 1 | 3 | 5 | +++ |
| | 10 | – | Toxic |

Experiment 3

In this experiment, the activity against experimental tumor Leukemia L-1210 in mice is tested.

Leukemia L-1210 cells were transplanted intraperitoneally (Number of transplanted cells was $10^6$ per mouse) to $CDF_1$ mice. The test compound was then administered intraperitoneally to the mice on the first, fifth and ninth day after transplantation. The effect of the test compound was evaluated by means of extension of the average survival period T/C % (average survival days of the groups administered with the test compound/average survival days of the control group). When T/C % is equal to or more than 120%, the test compound is considered to be effective.

The results are shown in Tables 6 and 7.

TABLE 6

| Compound No. | Toxic Dose (mg/kg) | Optimum Dose (mg/kg) | Optimum Dose T/C (%) | MED (mg/kg) | MED T/C (%) | T.I. |
|---|---|---|---|---|---|---|
| 1 | 12.5 | 3.12 | 189 | ≦0.78 | 156 | ≧4 |
| 2 | 100 | 50 | 212 (2) | 6.25 | 130 | 8 |
| 3 | ≧100 | 12.5 | 313 (4) | 1.56 | 136 | ≧8 |
| 4 | 50 | 25 | 313 (2) | 1.56 | 130 | 16 |
| 5 | 200 | 25 | 154 | 6.25 | 129 | 4 |
| 6 | 200 | 50 | 330 (2) | 6.25 | 141 | 8 |
| 7 | 50 | 25 | 274 (1) | 6.25 | 191 | 4 |

TABLE 7

| Compound No. | Dose (mg/kg) 100 | 50 | 25 | 12.5 | 6.25 | 3.12 | 1.56 | 0.78 | 0.39 |
|---|---|---|---|---|---|---|---|---|---|
| 1 | – | – | – | 0 | (165) | 189 | 152 | 156 | – |
| 2 | – | (212) (2) | 196 | 148 | 130 | 113 | 110 | – | – |
| 3 | – | (129) (1) | 284 (3) | 313 (4) | 198 | 167 | 136 | – | – |
| 4 | – | 79 | (313) (2) | 221 | 218 (1) | 154 | 130 | – | – |
| 5 | 90 | 139 | 154 | 125 | 129 | – | – | – | – |
| 6 | 98 | 330 (2) | 269 (1) | 213 (1) | 141 | 114 | – | – | – |
| 7 | 74 | 83 | 274 (1) | 212 (1) | 191 (1) | – | – | – | – |

MED: Minimum Effective Dose, the lowest dose when T/C % exceeds 120 %

T.I.: Therapeutic Index (Optimum Dose / MED)

Figures in parentheses are toxic by the decrease of body weight

Number in parentheses, right hand side below, indicates the survival to 6 mice

## Example 1

Cis-sulfato(trans-l-1,2-diaminocyclohexane) platinum(II) complex

In this example, 0.38 g (1 mmol) of $[Pt(dac)Cl_2]$ (dac is the, trans-l- -isomer) is added to a solution of 0.35 g of silver sulfate in 100 ml of water. The mixture is stirred at room temperature for 2 days under interception of light. AgCl formed is removed by filtration and to the filtrate there is added 5% (w/v) KCl solution, little by little, to remove $Ag^+$. The filtrate is dried at 50—60°C under reduced pressure using an evaporator whereby the desired sulfato-complex is obtained as a yellowish white powder.

Yield: 75% trans-l-complex

Example 2

Cis-nitrato-D-glucuronato(1,2-diaminocyclohexane) platinum(II) complex

In this example, 0.22 g (0.5 mmol) of [Pt(dac)$(NO_3)_2$] (dac is a cis-, trans-l- or trans-d-isomer) is dissolved in 10 ml of water with heating and the solution is cooled to room temperature. Then, 0.12 g (0.5 mmol) of sodium glucuronate is added to the solution and the mixture is allowed to stand at room temperature for one week. The solution is then dried at 50—60°C. under reduced pressure using an evaporator to obtain a yellow residue. The residue is washed with warm methanol to obtain the desired product.

Yield = 75% (cis-complex) and 70% (trans-l- or trans-d-complex).

[Pt(dac)$(NO_3)_2$] is prepared as follows:

1.14 g (3 mmol) of [Pt(dac)$Cl_2$] (dac is a cis-, trans-l- or trans-d-isomer) is suspended in 100 ml of water and to the suspension is added 1.02 g (3 mmol) of $AgNO_3$. The mixture is stirred at room temperature for 2 days under interception of light and the resultant AgCl is removed by filtration. Then, 5% (w/v) KCl solution is added to the filtrate little by little to remove unreacted $AgNO_3$. The solution free of $Ag^+$ is dried with heating under reduced pressure using an evaporator whereby the desired complexes are obtained as a yellowish white powder.

Yield: 80% (trans-l- or trans-d-complex) and 85% (cis-complex).

The [Pt(dac)$Cl_2$] used as a starting compound is prepared as follows:

An aqueous solution of 5 g of dac (a cis-, trans-l- or trans-d-isomer) and 18 g of $K_2(PtCl_4)$ is allowed to react at room temperature for about 12 hours to precipitate yellow needle-like crystals. The crystals are obtained by filtration and recrystallized from 0.1N HCl.

Yield: 73% (cis-complex) and 78% (trans-l- or trans-d-complex).

Example 3

Cis-bis-D-glucuronato(1,2-diaminocyclohexane) platinum(II) complex

In this example, 0.22 g (0.5 mmol) of [Pt(dac)$(NO_3)_2$] (dac is a cis-, trans-l- or trans-d-isomer) is dissolved in 10 ml of water with heating. After the solution is cooled to room temperature, 0.24 g (1 mmol) of sodium glucuronate is added thereto and the mixture is allowed to stand at room temperature for a week. The resultant solution is dried at 50—60°C under reduced pressure using an evaporator to obtain a yellow powder which is then washed with warm methanol and dried to obtain the desired product.

Yield: 70% (cis-, trans-l- or trans-d-complex).

Characteristic points of the infrared absorption spectra of the cis-platinum (II) complexes obtained in the above Examples are explained below.

Pt(dac)$(SO_4)$

Sulfato-complexes exhibit a broad absorption spectrum due to $\nu SO_4$ at 1120, 1030 and 950 $cm^{-1}$.

Geometrical isomers of dac are poorly distinguished by spectrum at 900—1000 $cm^{-1}$ because their peaks overlap that of $\nu SO_4$.

Pt(GlucH)$NO_3$(dac)

Peaks due to $NH_2$ and OH are observed respectively at 3100—3250 $cm^{-1}$ and 3300—3600 $cm^{-1}$. Peaks due to $\nu CO_2$(asym) and $\nu CO_2$(sym) are observed respectively at 1600 $cm^{-1}$ and 1400 $cm^{-1}$. Peaks due to $\nu NO_3$ are observed at 995, 1300 and 1500 $cm^{-1}$, whereby it is concluded that $NO_3^-$ is included in the complex.

Pt$(GlucH)_2$(dac)

A broad absorption band is observed at 3200—3400 $cm^{-1}$, which is attributed to the overlap of $\nu$ OH(GlucH) with $\nu NH_2$(dac). Peaks due to $\nu CO_2$(asym) and $\nu CO_2$(sym) are observed respectively at 1610 $cm^{-1}$ and 1400 $cm^{-1}$.

**Claims**

1. A cis-platinum (II) complex represented by the general formula

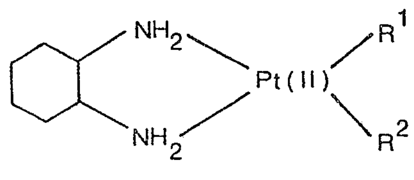

wherein $R^1$ and $R^2$ are the same or different and represent $NO_3^-$ or

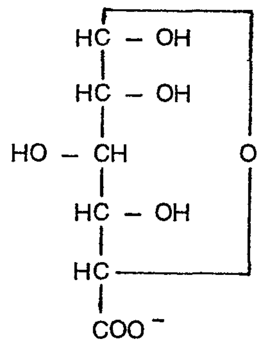

provided that $R^1$ and $R^2$ are not $NO_3^-$ at the same time or $R^1$ and $R^2$ combine to form $SO_4^{--}$ and the stereoisomerism of 1,2-diaminocyclohexane is cis-, trans-l- or trans-d-, provided that when $R^1$ and $R^2$ combine to form $SO_4^{--}$, the stereoisomerism of 1,2-diaminocyclohexane is trans-l-.

2. Compound according to claim 1, characterized in that it is cis-Pt(II) (trans-l-1,2-diaminocyclohexane)$SO_4$.

3. Compound according to claim 1, characterized in that it is Cis-Pt(II) (cis-1,2-diaminocyclohexane) (D-GlucH)$NO_3$ wherein GlucH means

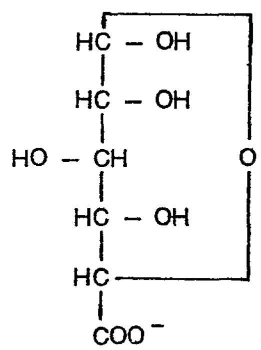

4. Compound according to claim 1, characterized in that it is cis-Pt(II) (trans-l-1,2-diaminocyclohexane) (D-GlucH)$NO_3$.

5. Compound according to claim 1, characterized in that it is cis-Pt(II) (trans-d-1,2-diaminocyclohexane) (D-GlucH)$NO_3$.

6. Compound according to claim 1, characterized in that it is cis-Pt(II) (cis-1,2-diaminocyclohexane) (D-GlucH)$_2$.

7. Compound according to claim 1, characterized in that it is cis-Pt(II) (trans-l-1,2-diaminocyclohexane) (D-GlucH)$_2$.

8. Compound according to claim 1, characterized in that it is cis-Pt(II) (trans-d-1,2-diaminocyclohexane) (D-GlucH)$_2$.

9. Pharmaceutical composition characterized in that it contains at least a cis-platinum II complex as defined in claim 1 to 8.

**Revendications**

1. Complexe cis de platine (II) représenté par la formule générale:

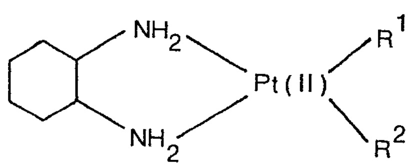

dans laquelle $R^1$ et $R^2$ sont identiques ou différents et représentent $NO_3^-$ ou

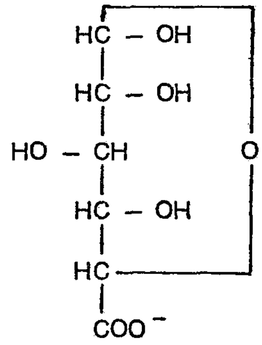

pourvu que $R^1$ et $R^2$ ne soient pas $NO_3^-$ en même temps, ou bein $R^1$ et $R^2$ se combinent pour former $SO_4^{--}$, et la stéréoisomérie du 1,2-diaminocyclohexane est cis-, trans-l- ou trans-d-, pourvu que lorsque $R^1$ et $R^2$ se combinent pour former $SO_4^{--}$, la stéréo-isomérie du 1,2-diaminocyclohexane soit trans-l.

2. Composé selon la revendication 1, caractérisé par le fait que c'est le cis-Pt(II) (trans-l-1,2-diaminocyclohexane)$SO_4$.

3. Composé selon la revendication 1, caractérisé par le fait que c'est le cis-Pt(II) (cis-1,2-diaminocyclohexane) (D-GlucH)$NO_3$ dans laquelle GlucH désigne

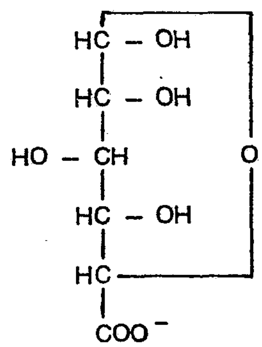

4. Composé selon la revendication 1, caractérisé par le fait que c'est le cis-Pt(II) (trans-l-1,2-diaminocyclohexane) (D-GlucH)$NO_3$.

5. Composé selon la revendication 1, caractérisé par le fait que c'est le cis-Pt(II) (trans-d-1,2-diaminocyclohexane) (D-GlucH)$NO_3$.

6. Composé selon la revendication 1, caractérisé par le fait que c'est le cis-Pt(II) (cis-1,2-diaminocyclohexane) $(D\text{-}GlucH)_2$.

7. Composé selon la revendication 1, caractérisé par le fait que c'est le cis-Pt(II) (trans-l-1,2-diaminocyclohexane) $(D\text{-}GlucH)_2$.

8. Composé selon la revendication 1, caractérisé par le fait que c'est le cis-Pt(II) (trans-d-1,2-diaminocyclohexane) $(D\text{-}GlucH)_2$.

9. Composition pharmaceutique, caractérisée par le fait qu'elle contient au moins un complexe cis de platine II tel que défini dans les revendications 1 à 8.

**Patentansprüche**

1. Ein cis-Platin(II)-Komplex der allgemeinen Formel

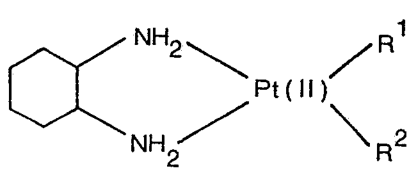

in dem der $R^1$ und $R^2$ gleich oder unterschiedlich sind und $NO_3^-$ oder

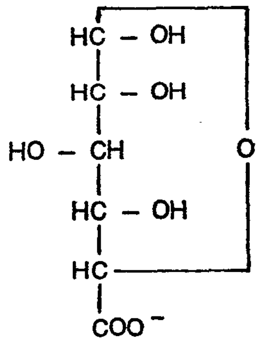

sofern $R^1$ und $R^2$ nicht gleichzeitig $NO_3$ sind, darstellen, oder $R^1$ und $R^2$ sich zu $SO_4^{--}$ zusammenschliessen und der Stereoisomerismus des 1,2-diaminocyclohexan cis-, trans-l- oder trans-d-, ist, wobei, wenn $R_1$ und $R_2$ sich zu $SO_4^{--}$ zusammenschliessen, der Stereoisomerismus des 1,2-diaminocyclohexan trans-l- ist.

2. Verbindung gemäss Anspruch 1, dadurch gekennzeichnet, dass es sich um cis-Pt(II) (trans-l-1,2-diaminocyclohexan)$SO_4$ handelt.

3. Verbindung gemäss Anspruch 1, dadurch gekennzeichnet, dass es sich um cis-Pt(II) (cis-1,2-diaminocyclohexan) (D-GlucH)$NO_3$ handelt, in dem GlucH bedeutet:

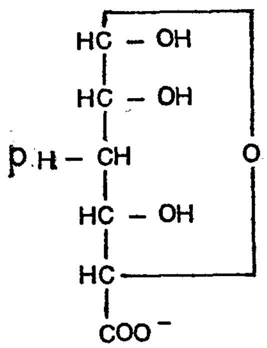

4. Verbindung gemäss Anspruch 1, dadurch gekennzeichnet, dass es sich um cis-Pt(II) (trans-l-1,2-diaminocyclohexan) (D-GlucH)$NO_3$ handelt.

5. Verbindung gemäss Anspruch 1, dadurch gekennzeichnet, dass es sich um cis-Pt(II) (trans-d-1,2-diaminocyclohexan) (D-GlucH)$NO_3$ handelt.

6. Verbindung gemäss Anspruch 1, dadurch gekennzeichnet, dass es sich um cis-Pt(II) (cis-1,2-diaminocyclohexan) $(D\text{-}GlucH)_2$ handelt.

7. Verbindung gem. Anspruch 1, dadurch gekennzeichnet, dass es sich um cis-Pt(II) (trans-l-1,2-diaminocyclohexan) $(D\text{-}GlucH)_2$ handelt.

8. Verbindung gemäss Anspruch 1, dadurch gekennzeichnet, dass es sich um cis-Pt(II) (trans-d-1,2-diaminocyclohexan) $(D\text{-}GlucH)_2$ handelt.

9. Heilmittel, dadurch gekennzeichnet, dass es mindestens einen cis-Plantin(II)-Komplex gemäss obigen Ansprüchen 1 bis 8 enthält.